# EUROPEAN PATENT APPLICATION

(11) **EP 1 103 224 A1**
(43) Date of publication of application: **30.05.2001**
(21) Application number: 00310461.9
(22) Date of filing: 24.11.2000
(51) Int. Cl.: A61B 17/12

(54) **Vascular occluder**

(30) Priority: 24.11.1999 GB 9927711
(71) Applicant: Robinson, Nicholas, Hereford HR4 9QB (GB)
(72) Inventor: Robinson, Nicholas, Hereford HR4 9QB (GB)
(74) Representative: Goodenough, Nigel

(57) **Abstract**

Vascular occluder, for blocking severed or opened vascular vessels, comprising:
a rod (16) having an expanded, rounded tip (18);
a resiliently flexible balloon (12) which encloses said tip. The balloon is attached to an elongate sleeve (10) which substantially encloses at least part of the rod.

The rod is free to move axially with respect to the balloon and sleeve,

In use, the rod is inserted into or removed from a vascular vessel with the tip pressing against an internal surface of the balloon so as to reduce the balloon's outer diameter. When the occluder is within a vascular vessel, the tip can be moved away from the internal surface so as to allow the balloon to automatically expand in diameter to occlude the vascular vessel.

## Description

This invention relates to the field of vascular occluders, in particular those suitable for use during coronary bypass surgery.

Vascular occluders are used during surgery to stop excess bleeding from veins and arteries that have been opened or severed during the process of surgery.

US-A-4946463 (Pioneering Technologies Inc) describes a vessel occluder suitable for use in coronary artery surgery. The occluder comprises two smooth bullet-shaped occluders, thermo-formed from polyurethane tubing. The occluders are each fixed to the end of a mono-filament, which is looped and passed through a length of radiopaque soft plastic tubing which acts as a simple holder.

In use, the bullet-shaped occluders are firmly inserted into the lumen of the artery to be occluded. The occluder prevents haemorrhage from the artery and serves to hold the vessel in a circular configuration. However, this device has the disadvantage that it is necessary to provide a range of sizes of the bullet-shaped occluders and the surgeon will need to make a judgement as to the appropriate size of occluder to be used. The occluders are supplied in diameters varying by 0.25mm increments and it is therefore likely that, in some cases, an occluder of larger than ideal diameter will have to be employed. In such cases, there is a risk of damaging the vessel wall on insertion of the occluder, which may be slightly too large for that particular vessel. In any event, grasping and squeezing artery walls to insert a close fitting occluder is potentially traumatic for the artery and may damage its delicate endothelial layer.

Furthermore, even in normal use, the bullet-shaped occluders are relatively hard and necessarily have to provide a tight fit within the vessel in order to function correctly. There is always, therefore, the risk of damage to the walls of veins or arteries when the occluders are inserted or removed.

There is thus a need for a vascular occluder which reduces the risk of damage to the vessel in which it is employed It is an object of the present invention to provide such a vascular occluder. It is also an object to provide an occluder which fits more sizes of artery so that a smaller number of sizes of them are needed, and hence their overall cost can be reduced.

According to the present invention, there is provided a vascular occluder, for blocking severed or opened vascular vessels, comprising:
a sleeve;
a rod slidable within the sleeve and longer than the sleeve; and
a resiliently flexible balloon formed on one end of the tube and having a normal diameter which is reduced when the rod is pushed through the sleeve to impinge on the inside of the balloon and stretch the balloon longitudinally.

Preferably, the rod has a rounded tip to reduce the possibility of puncturing the balloon.

In use, therefore, the occluder is inserted into or removed from a vascular vessel with said tip pressing against the internal surface of the balloon so as to reduce the balloon's outer diameter. Then, when the occluder is within a vascular vessel, the rod can be withdrawn so as to allow the balloon to resiliently expand in diameter to occlude the vascular vessel.

Preferably, the resilience of the balloon is sufficient to overcome resistance to movement of the rod in the sleeve, other than user imposed resistance.

A vascular occluder according to the present invention has the advantage that, by being able to vary the diameter of the balloon, the device can adjust automatically to occlude differently-sized veins or arteries. This reduces the risk of damage to the vascular vessel resulting from an incorrectly-sized occluder and also eliminates the need to provide a large range of occluders of varying sizes.

Preferably, said rod further comprises a handle at an end distal from said tip. This facilitates gripping of the device, in use.

Preferably, said rod is semi-rigid.

Preferably, said balloon has a thickened internal surface against which said tip presses, in use. This reduces the likelihood of the tip undesirably penetrating the internal surface of said balloon.

Preferably, said tip is sufficiently large to trap said rod within said sleeve. This enables the rod and balloon to be conveniently kept together so that they do not become separated.

Preferably, said balloon has a normally ellipsoidal or ovoidal shape. Alternatively, it may comprise the shape essentially of two cones placed base to base, so that a discrete seal edge is defined.

A preferred embodiment of the invention is now described, by way of example, with reference to the accompanying drawings in which:

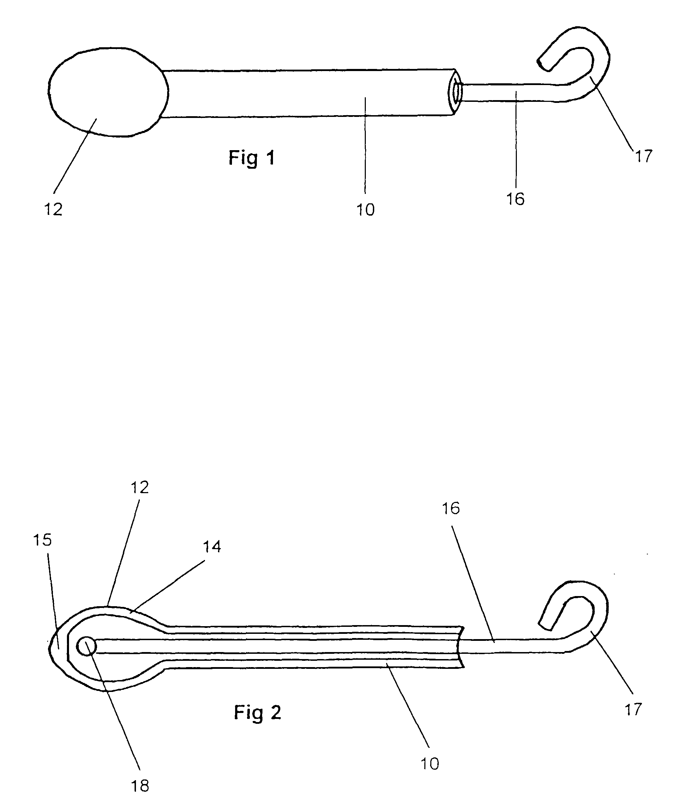

Figure 1 shows in profile a vascular occluder embodying the present invention; and
Figure 2 shows in section the vascular occluder of Figure 1.

Referring to the drawings, the vascular occluder comprises a tube or elongate sleeve 10 attached firmly to a surgical balloon 12 which has flexible walls 14 and a thickened front section 15. The balloon is made of a resilient rubber or elastomeric material such as silicone rubber. The sleeve is conveniently made from PTFE medically etched tube.

Inside the sleeve 10 there is a rod 16 of stainless steel wire, which is formed with a handle 17 and an expanded rounded, tip 18. The tip might be formed directly from the wire of the rod 16, but conveniently it is formed simply by a drop of epoxy resin glue or the like. The expanded tip 18 prevents puncturing the surgical balloon 12 when the rod 16 is pushed forward to thin down the profile of the surgical balloon 12. The expanded tip 18 can also be used to trap the rod 16 within the tube 10 so that they do not get separated.

In operation, the tube 10 is used to present the surgical balloon 12 against the wound in the artery or vein. The handle 17 is then used to push the rod 16 axially against the internal surface of the surgical balloon 12. This thins down the profile, ie reduces the outer diameter of the surgical balloon 12, which means it can be inserted into the vein or artery without damaging the vein or artery walls. The soft composition of the surgical balloon 12 also helps to prevent damage to the walls of the vein or artery during insertion.

When the surgical balloon 12 is in the desired location within the artery or vein, the handle 17 is used to withdraw the rod 16 axially away from the internal surface of the surgical balloon 12. On the other hand, the natural resilience of the balloon material will push the rod along the sleeve unless the rod is held in position by the user. In any event, this allows the surgical balloon 12 to expand in diameter until its flexible walls 14 abut the internal walls of the artery or vein and create a seal. Thus, the balloon 12 automatically adjusts to the correct diameter, which not only reduces the likelihood of damage through using an occluder which is too large, but also eliminates the need to provide a whole set of occluders of varying diameters. Furthermore, the soft silicone rubber only gently presses against the internal vascular walls and has little likelihood of damaging the endothelial layer of arteries or veins in which occluder is operational.

To remove the vascular occluder, the tube 10 is grasped and the rod 16 is slid forward to thin the surgical balloon, that is, to reduce its outer diameter again. This allows the vascular occluder to be extracted without damaging the walls of the vein or artery.

The balloon 14 is normally ellipsoidal in shape, although it may also be more conically shaped to define a more discrete seal edge when in its normal, unstretched condition.

The sleeve and/or the rod may be rigid or semi-rigid or flexible as required.

## Claims

1. Vascular occluder, for blocking severed or opened vascular vessels, comprising:
a sleeve;
a rod slidable within the sleeve and longer than the sleeve; and
a resiliently flexible balloon formed on one end of the tube and having a normal diameter which is reduced when the rod is pushed through the sleeve to impinge on the inside of the balloon and stretch the balloon longitudinally.

2. Vascular occluder as claimed in claim 1 wherein said rod has a rounded tip to reduce the possibility of puncturing the balloon.

3. Vascular occluder as claimed in claim 2, wherein said tip is sufficiently large to trap said rod within said sleeve.

4. Vascular occluder as claimed in claim 2 or 3, wherein said rod further comprises a handle at an end distal from said tip.

5. Vascular occluder as claimed in any preceding claim, wherein said rod is semi-rigid, preferably of steel wire.

6. Vascular occluder as claimed in any of the preceding claims wherein said balloon has a thickened internal surface against which said rod presses, in use.

7. Vascular occluder as claimed in any of the preceding claims wherein said balloon is made from silicone rubber.

8. Vascular occluder as claimed in any of the preceding claims wherein said balloon has a normally ellipsoidal or ovoidal shape.

9. Vascular occluder as claimed in any of the preceding claims, wherein the sleeve is contructed from medically etched PTFE.
